(19) Europäisches Patentamt
European Patent Office
Office européen des brevets

(11) Veröffentlichungsnummer: **0 492 119 B1**

# (12) EUROPÄISCHE PATENTSCHRIFT

(45) Veröffentlichungstag der Patentschrift: **28.12.94**

(51) Int. Cl.$^{5}$: **A61K 7/09**

(21) Anmeldenummer: **91119487.6**

(22) Anmeldetag: **15.11.91**

## (54) Verfahren zur dauerhaften Verformung von Haaren.

(30) Priorität: **21.12.90 DE 4041164**

(43) Veröffentlichungstag der Anmeldung:
**01.07.92 Patentblatt 92/27**

(45) Bekanntmachung des Hinweises auf die Patenterteilung:
**28.12.94 Patentblatt 94/52**

(84) Benannte Vertragsstaaten:
**DE ES FR GB IT**

(56) Entgegenhaltungen:
**EP-A- 0 346 151**
**DE-A- 1 492 174**
**FR-A- 2 162 647**
**GB-A- 672 838**
**GB-A- 1 423 342**

(73) Patentinhaber: **Wella Aktiengesellschaft**
**Berliner Allee 65**
**D-64295 Darmstadt (DE)**

(72) Erfinder: **Schwan, Annette, Dr.**
**Scheppallee 57**
**W-6100 Darmstadt (DE)**
Erfinder: **Lang, Gònther, Dr.**
**Auf der Roten Erde 10 B**
**W-6107 Reinheim 5 (DE)**
Erfinder: **Clausen, Thomas, Dr.**
**Ernst-Pasque-Str. 35 A**
**W-6146 Alsbach (DE)**

**EP 0 492 119 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur dauerhaften Haarverformung, bei dem das Haar zunächst mit einem ein Bunte-Salz und ein Sulfit oder Hydrogensulfit enthaltenden Verformungsmittel verformt wird und anschließend mit einem Oxidationsmittel behandelt wird.

Die dauerhafte Verformung von Haaren erfolgt in der Regel, indem das Haar zunächst mit einer keratinreduzierenden Thiolverbindung, wie zum Beispiel einem Mercaptocarbonsäuresalz oder einem Mercaptocarbonsäureester, behandelt wird, sodann in seine neue Form gebracht wird und anschließend mit einer Oxidationsmittellösung oxidativ nachbehandelt ("fixiert") wird.

Diese Vorgehensweise ist jedoch mit einigen Nachteilen verbunden. So besitzt das Haar häufig nach der Behandlung einen unangenehmen Mercaptangeruch, der in einigen Fällen noch Tage nach der Verformungsbehandlung wahrnehmbar ist. Weiterhin können bei Verwendung von Mercaptocarbonsäureestern allergische Reaktionen auftreten, während bei Verwendung von Mercaptocarbonsäuresalzen aufgrund des hohen pH-Wertes des Verformungsmittels Haarschäden nicht ausgeschlossen werden können.

Es wurde deshalb schon mehrfach vorgeschlagen, für die Verformungsbehandlung anstelle von thiolhaltigen Reduktionsmitteln sulfithaltige Mittel zu verwenden, da sulfithaltige Verformungsmittel gegenüber thiolhaltigen Mitteln Vorteile aufweisen. So weisen sulfithaltige Verformungsmittel keinen störenden Eigengeruch auf, sind nicht sensibilisierend und besitzen zudem bereits im neutralen pH-Bereich ihre volle Verformungswirkung, wodurch eine haar- und hautschonende Verformungsbehandlung ermöglicht wird.

Ein Nachteil der Haarverformung mit sulfithaltigen Mitteln ist es jedoch, daß sich das Haarkeratin nach der Verformungsbehandlung nur schwer stabilisieren läßt (das heißt eine Wiederherstellung des ursprünglichen Zustandes der Haare nicht möglich ist ), da die bei Verwendung von thiolhaltigen Verformungsmitteln üblicherweise verwendeten oxidative Nachbehandlung bei sulfitverformtem Haar keine ausreichende Stabilisierung bewirkt und die Stabilisierung durch intensives Spülen mit Wasser ebenfalls nicht befriedigend ist.

Aus diesem Grunde besitzt eine mit sulfithaltigen Verformungsmitteln erzielte Haarverformung nur eine mäßige Haltbarkeit. Zudem weist das so behandelte Haar einen flattrigen und rauhen Griff auf.

In der EP-OS 0 346 151 wird deshalb vorgeschlagen, zur Haarverformung alkalische Zubereitungen zu verwenden, die neben einem Alkali- oder Ammoniumsulfit zusätzlich ein Bunte-Salz enthalten. Dieses Bunte-Salz soll, vor dem stabilisierenden Spülschritt mit Wasser, unter Abschirmung der feuchten Haare mit einer Folie thermisch am Haar verankert werden. Hierdurch soll eine Verbesserung der Haltbarkeit der Haarverformung erzielt werden, da das Bunte-Salz mit den Thiolgruppen des Haarkeratins reagiert und so eine erhöhte Vernetzung am Haar bewirkt. Eine oxidative Nachbehandlung erfolgt nicht.

Dieses Verfahren besitzt jedoch den Nachteil, daß es sehr Zeitaufwendig ist, da jeder Dauerwellwickler in eine separate Folie eingewickelt werden muß, um ein befriedigendes Wellergebnis zu erzielen. Das bei diesem Verfahren eingesetzte Verformungsmittel weist zudem einen sehr hohen pH-Wert (pH > 10) auf, wodurch das Haar übermäßig stark beansprucht wird. Aufgrund der starken Beanspruchung des Haarkeratins besitzt das auf diese Weise behandelte Haar einen schlechten Griff und eine mangelhafte Frisierbarkeit in trockenem Zustand.

Aufgabe der vorliegenden Erfindung ist es daher, ein Verfahren zur dauerhaften Verformung von Haaren zur Verfügung zu stellen, das sowohl eine haar- und hautschonende Verformungsbehandlung ermöglicht als auch eine gute Haltbarkeit der Verformung gewährleistet. Weiterhin sollen einerseits die Haare nach der Verformungsbehandlung einen guten Griff aufweisen und frei von störendem Mercaptangeruch sein sowie andererseits die verwendeten Verformungsmittel nicht sensibilisierend sein.

Es wurde nun überraschenderweise gefunden, daß diese Aufgabe durch ein Verfahren zur dauerhaften Haarverformung, bei dem das Haar mit einem Verformungsmittel, welches ein Sulfit oder Hydrogensulfit sowie ein Bunte-Salz enthält, verformt wird und anschließend oxidativ nachbehandelt wird, in hervorragender Weise gelöst wird.

Gegenstand der vorliegenden Erfindung ist daher ein Verfahren zur dauerhaften Verformung von Haaren, bei dem man das Haar bevor und/oder nachdem man es in die gewünschte Form bringt mit einem Verformungsmittel behandelt, gegebenenfalls mit Wasser spült, sodann oxidativ nachbehandelt, mit Wasser spült, gegebenenfalls zur Wasserwelle legt, und sodann trocknet, welches dadurch gekennzeichnet ist, daß man als Verformungsmittel eine wäßrige Zubereitung, welche mindestens ein Sulfit oder Hydrogensulfit sowie mindestens ein BunteSalz enthält, verwendet.

Bei einer Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von etwa 5 bis 15 Millimetern gewickelt, sodann wird auf das Haar eine für die Haarverformung ausreichende Menge, vorzugsweise etwa 80 bis 120 Gramm, des Verformungsmittels aufgetragen.

In einer weiteren Ausführungsform des erfindungsgemäßen Verfahrens wird das handtuchtrockene Haar mit einem Teil des Verformungsmittels, vorzugsweise etwa 40 bis 60 Gramm, vorgefeuchtet, in einzelne Strähnen aufgeteilt und auf Wickler gewickelt. Sodann wird auf das Haar das restliche Verformungsmittel, vorzugsweise etwa 40 bis 60 Gramm, aufgetragen.

Das bei dem hier beschriebenen Verfahren verwendete Verformungsmittel ist eine wäßrige Zubereitung, welche eine Mischung aus einem oder mehreren Sulfiten oder Hydrogensulfiten und einem oder mehreren Bunte-Salzen enthält.

Als Sulfite oder Hydrogensulfite werden vorzugsweise Alkalisulfite oder -hydrogensulfite, beispielsweise Natriumsulfit oder -hydrogensulfit, Ammoniumsulfit oder -hydrogensulfit und Alkanolaminsalze der schwefligen Säure in einer Menge von 0,5 bis 30 Gewichtsprozent, vorzugsweise in einer Menge von 2 bis 12 Gewichtsprozent, verwendet.

Für die Verwendung in dem Verformungsmittel des hier beschriebenen Verfahrens sind beispielsweise die folgenden monomeren oder polymeren Bunte-Salze oder Mischungen dieser Bunte-Salze geeignet:

(i) 2-S-Thiosulfatocarbonsäuren der allgemeinen Formel (I)

$$M^{\oplus}\,{}^{\ominus}OOC\text{-}(CH_2)_n\text{-}S_2O_3{}^{\ominus}M^{\oplus} \qquad (I),$$

mit n gleich einer ganzen Zahl von 1 bis 6 und $M^{\oplus}$ gleich einem Alkalimetall- oder Ammoniumion;

(ii) 2-S-Thiosulfatoethylamine der allgemeinen Formel (II)

$$H_2N\text{-}CH_2\text{-}CH_2\text{-}S_2O_3{}^{\ominus}\ M^{\oplus} \qquad (II),$$

mit $M^{\oplus}$ gleich einem Alkalimetall- oder Ammoniumion;

(iii) Polyamid-Epichlorhydrin-Bunte-Salze der allgemeinen Formel (III)

$$P\text{-}N\text{-}CH_2\text{-}CH(OH)\text{-}CH_2\text{-}S_2O_3{}^{\ominus}\ M^{\oplus} \qquad (III),$$

mit $M^{\oplus}$ gleich einem Alkalimetall- oder Ammoniumion und P = Polymerkette;

(iv) Polyoxyalkylen-Bunte-Salze der allgemeinen Formel (IV) oder (V)

$$\left[\,R\,\right] \begin{array}{l} \left[(O\text{-}Alkylen)_m\ OH\right]_q \\ \left[(O\text{-}Alkylen)_m\ O\ X\ S_2O_3Y\right]_p \end{array} \qquad (IV)$$

$$YO_3S_2\ X\ \text{-}\ (O\text{-}Alkylen)_m\ \text{-}\ X\ S_2O_3Y \qquad (V),$$

wobei
p = 2, 3, 4, 5 oder 6;
q = 0, 1, 2, 3 oder 4;
(p + q) = 3, 4, 5 oder 6;
m gleich einer ganzen Zahl ≧ 1;
R einen Rest darstellt, der durch Abspalten der Hydroxylgruppen aus einem aliphatischen Alkohol mit mindestens 2 Kohlenstoffatomen erhalten wird;

X ein zweiwertiger substituierter oder unsubstituierter aliphatischer Rest mit 1 bis 10 Kohlenstoffatomen ist ; und

Y ein Wasserstoffatom, ein salzbildendes Ion oder eine salzbildende Gruppe darstellt;

(v) (3-S-Thiosulfato-2-hydroxy-propyl)-H,N-dimethylamin der allgemeinen Formel (VI)

$$(CH_3)_2N\text{-}CH_2\text{-}CH(OH)\text{-}CH_2\text{-}S_2O_3 \qquad (VI);$$

(vi) durch Umsetzung von Keratinhydrolysaten (beispielsweise Kerasol® der Firma Croda Ltd/Großbritannien) mit Natriumthiosulfat erhaltene Keratin-Bunte-Salze;

(vii) Polysiloxan-Bunte-Salze der allgemeinen Formel (VII)

$$\underset{\displaystyle R^2_b}{R^1_a\text{-}\underset{|}{Si}}\text{-}O_{\frac{4-(a+b)}{2}} \quad (VII)$$

wobei

- $R^1$ = Methyl, jedoch bis zu 10 % der Gruppe $R^1$ Phenylreste sein können,
- $R^2$ = $R^3$ und/oder $R^4$

mit

- $R^3$ =

$$-(C_nH_{2n})-O-(C_mH_{2m}O)_p-R^5_q-CH_2\overset{R^7}{\overset{|}{C}}HCH_2R^6$$

- $R^5$ =

$$-\underset{CH_2Halogen}{\underset{|}{C}H}-CH_2O- \quad \text{oder} \quad -CH_2-\underset{CH_2Halogen}{\underset{|}{C}H}-O-$$

- $R^6$,$R^7$ = eine der beiden Gruppen -OH, die andere $-S_2O_3Me$,
- Me = Alkalimetall oder gegebenenfalls substituierter Ammoniumrest,
- n = 3 bis 6,
- m = 2 bis 3,
- p = 0 bis 100,
- q = 0 bis 5,

und

- $R^4$ = $-(C_xH_{2x})-O-(C_yH_{2y}0)_zR^8$
- $R^8$ = Wasserstoff, Alkylrest mit 1 bis 4 Kohlenstoffatomen oder Acetylrest,
- x = 3 bis 6,
- y = 2 bis 3,
- z = 1 bis 100, ist,

mit der Maßgabe, daß im durchschnittlichen Molekül mindestens ein Rest $R^2$ die Bedeutung des Restes $R^3$ hat, und wenn im Rest $R^3$ p = 0 ist, mindestens 1 Rest $R^4$ vorhanden ist,

- a = 1 bis 2,33
- b = 0,02 bis 1 und
- $2 \leq (a + b) \leq 3$ ist.

Vorzugsweise werden Bunte-Salze der allgemeinen Formel (I), (III), (VI) oder (VII) sowie Keratin-Bunte-Salze verwendet, wobei Bunte-Salze der Formel (VI), der Formel (III) mit P = Adipinsäure-Trithylendiamin-Polyamid-Epichlorhydrin-Harz (Hercosett® 57 oder 125 der Hercules Inc./USA) sowie Keratin-Bunte-Salz besonders bevorzugt sind.

Die Bunte-Salze werden in dem Verformungsmittel in einer Menge von 0,1 bis 30 Gewichtsprozent, vorzugsweise in einer Menge von 0,5 bis 15 Gewichtsprozent, verwendet.

Der pH-Wert des in dem erfindungsgemäßen Verfahren verwendeten Verformungsmittels beträgt 6 bis 10, wobei ein pH-Wert unter 9, insbesondere ein pH-Wert von 6 bis 8,5, bevorzugt ist.

Zur Wirkungssteigerung können dem Verformungsmittel Quell- und Penetrationsstoffe, wie zum Beispiel Dipropylenglykolmonomethylether, Harnstoff, 2-Pyrrolidon oder Imidazolidin-2-on, in einer Menge von etwa

2 bis 30 Gewichtsprozent zugesetzt werden.

Nach einer für die dauerhafte Verformung der Haare ausreichenden Einwirkungszeit, welche je nach Haarbeschaffenheit, dem pH-Wert und der Verformungswirksamkeit des Verformungsmittels sowie in Abhängigkeit von der Anwendungstemperatur (15 bis 80 Grad Celsius) 10 bis 60 Minuten (10 bis 30 Minuten mit Wärmeeinwirkung und 20 bis 60 Minuten ohne Wärmeeinwirkung) beträgt, wird das Haar mit Wasser gespült und sodann oxidativ nachbehandelt ("fixiert"). Es ist jedoch ebenfalls möglich, die oxidative Nachbehandlung ohne vorheriges Spülen mit Wasser direkt im Anschluß an die Verformungsbehandlung durchzuführen. Das Mittel zur oxidativen Nachbehandlung wird je nach Haarfülle, in einer Menge von etwa 80 bis 120 Gramm verwendet.

Gemäß einer weiteren Ausführungsform des erfindungsgemäßen Verfahrens kann das Haar vor der oxidativen Nachbehandlung auch getrocknet werden.

Für die oxidative Nachbehandlung kann jedes beliebige bisher für eine derartige Behandlung verwendete Mittel verwendet werden. Beispiele für in solchen Mitteln verwendbare physiologisch verträgliche Oxidationsmittel sind Alkalibromate, zum Beispiel Natrium- oder Kaliumbromat, Natriumperborat, Harnstoffperoxid und Wasserstoffperoxid. Das zur oxidativen Nachbehandlung verwendete Mittel liegt in Form einer wäßrigen Zubereitung vor und enthält üblicherweise 0,5 bis 10 Gewichtsprozent des physiologisch verträglichen Oxidationsmittels. Weiterhin kann das zur oxidativen Nachbehandlung verwendete Mittel weitere Stoffe, wie zum Beispiel schwache Säuren, Puffersubstanzen oder Peroxidstabilisatoren, enthalten. Die Einwirkungszeit des für die oxidative Nachbehandlung verwendeten Mittels beträgt in der Regel etwa 1 bis 20 Minuten.

Sowohl das bei dem erfindungsgemäßen Verfahren verwendete Verformungsmittel als auch das Mittel zur oxidativen Nachbehandlung kann in Form einer wäßrigen Lösung oder einer Emulsion sowie in verdickter Form auf wäßriger Basis, insbesondere als Creme, Gel oder Paste, vorliegen. Ebenfalls ist es möglich dieses Mittel unter Druck in Aerosoldosen abzufüllen und daraus als Schaum zu entnehmen. Weiterhin können sowohl das Mittel zur oxidativen Nachbehandlung als auch das Haarverformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl; ferner Farbstoffe; Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle; haarkonditionierende bzw. haarpflegende Bestandteile, wie zum Beispiel Lanolinderivate, Cholesterin, Pantothensäure oder Betain, enthalten. Die erwähnten Bestandteile werden in den für solche Zwecke üblichen Mengen verwendet, zum Beispiel die Verdickungsmittel in einer Menge von etwa 0,1 bis 25 Gewichtsprozent.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Durch das vorstehend beschriebene Verfahren zur Verformung von Haaren wird eine schonende und gleichmäßige, natürlich wirkende Umformung der Haare vom Haaransatz bis zu den Haarspitzen ermöglicht. Das so verformte Haar besitzt einen angenehmen Griff sowie eine gute Haltbarkeit der Frisur und ist frei von störendem Geruch.

Die nachfolgenden Beispiele sollen den Gegenstand der Erfindung erläutern.

## Beispiele

### Beispiel 1: Verfahren zur dauerhaften Haarverformung

Im Anschluß an eine Haarwäsche wird das handtuchtrockene Haar auf Wickler mit einem Durchmesser von 5 Millimetern gewickelt und mit 80 g des folgenden Haarverformungsmittels durchfeuchtet.

6,0 g Polyamid-Epichlorhydrin-Bunte-Salz der allgemeinen Formel (III) erhalten durch Umsetzung von Natriumthiosulfat mit einem Polyamid-Epichlorhydrin ("Hercosett® 125" der Fa. Hercules Inc., Wilmington/USA)
2,0 g Ammoniumsulfit
0,2 g Parfümöl
91,8 g Wasser
100,00 g (mit Natronlauge auf pH 10 eingestellt)

Nach einer Einwirkungszeit von 30 Minuten bei 50 Grad Celsius wird das Haar mit Wasser gründlich gespült und sodann mit 100 g einer wäßrigen Natriumbromatlösung der folgenden Zusammensetzung oxidativ nachbehandelt ("fixiert").

6,0 g Natriumbromat
1,0 g Dinatriumhydrogenphosphat
0,7 g Natriumdihydrogenphosphat
92,3 g Wasser
100,00 g (pH = 6,2)

Nach Entfernung der Wickler werden die Haare erneut mit Wasser gespült und anschließend getrocknet.

Das so behandelte Haar weist eine gute Verformung auf, die auch nach mehrmaliger Haarwäsche erhalten bleibt.

**Beispiel 2: Verfahren zur dauerhaften Haarverformung**

Auf das handtuchtrockene Haar wird etwa die Hälfte des nachstehenden Haarverformungsmittels verteilt, anschließend werden die Haare auf Wickler mit einem Durchmesser von 10 Millimetern gewickelt und sodann mit dem restlichen Haarverformungsmittel nochmals befeuchtet.

3,0 g Keratin-Bunte-Salz, erhalten durch Umsetzung von Natriumthiosulfat mit einem Keratinhydrolysat ("Kerasol®" der Fa. Croda Ltd./Großbritannien)
10,0 g Ammoniumhydrogensulfit
20,0 g Harstoff
4,0 g Ethanol
0,4 g Kokosalkyldimethylammoniumbetain
0,3 g Parfümöl
0,2 g hydriertes Rizinusöl, mit 45 Mol Ethylenoxid oxethyliert
62,1 g Wasser
100,0 g (mit Natronlauge auf pH 7,0 eingestellt)

Nach einer Einwirkungszeit von 20 Minuten bei 45 Grad Celsius, während der das Haar mit einer Plastikhaube abgedeckt wird, wird das Haar 3 Minuten lang mit lauwarmen Wasser gespült und sodann mit 80 g einer 3-prozentigen wäßrigen Wasserstoffperoxidlösung (pH = 2,5) oxidativ nachbehandelt. Anschließend werden die Wickler entfernt, die Haare erneut mit lauwarmen Wasser gepült, zur Wasserwelle gelegt und sodann getrocknet.

Das so dauerverformte Haar ist im nassen und trockenem Zustand leicht kämmbar und besitzt einen angenehmen Griff.

**Beispiel 3: Verfahren zur dauerhaften Haarverformung**

Im Anschluß an eine Haarwäsche wird das handtuchtrockene Haar auf Wickler mit einem Durchmesser von 8 Millimetern gewickelt und mit dem folgenden Haarverformungsmittel befeuchtet.

10,0 g (3-S-Thiosulfato-2-hydroxy-propyl)-N,N-dimethylamin der allgemeinen Formel (VI)
6,0 g Natriumsulfit
5,0 g 2-Pyrrolidon
4,0 g Isopropanol
0,4 g Parfümöl
0,4 g Octylphenol, mit 20 Mol Ethylenoxid oxethyliert
0,2 g Kokosalkyldimethylammoniumbetain
74,0 g Wasser
100,00 g (mit Natronlauge auf pH 8 eingestellt)

Das Haar wird sodann mit einer Plastikhaube abgedeckt und 20 Minuten auf 45 Grad Celsius erwärmt. Anschließend wird das Haar gründlich mit Wasser gespült und sodann 10 Minuten bei 50 Grad Celsius getrocknet.

Sodann wird das Haar in der in Beispiel 1 beschriebenen Weise oxidativ nachbehandelt, zur Wasserwelle gelegt und getrocknet.

Das so behandelte Haar weist eine hohe Wellstabilität auf, ist leicht kämmbar und besitzt einen angenehmen Griff.

**Beispiel 4: Vergleichsversuche**

**(a) Haltbarkeit der erzeugten Dauervelle in Abhängigkeit von der oxidativen Nachbehandlung**

Zwei feuchte Haarsträhnenaus jeweils 100 gebleichten menschlichen Haaren wurden unter Zug auf je einen Spiralwickler mit einem Durchmesser von 5 mm gewickelt und sodann mit 0,1 g eines der beiden

folgenden Verformungsmittel getränkt.

| Verformungsmittel | (A) | (B) |
|---|---|---|
| Bunte-Salze der Formel (III) erhalten durch Umsetzung eines Polyamid-Epichlorhydrins ("Hercosett® 125" der Fa. Hercules Inc., Wilmington/ USA) mit Natriumthiosulfat | 6,0 g | 6,0 g |
| Natriumsulfit | 2,0 g | 5,0 g |
| Wasser | ad 100,0 g | ad 100,0 g |
| | (mit Natronlauge auf pH = 10 eingestellt) | |

Nach einer Einwirkungszeit von 30 Minuten bei 50 Grad Celsius wurden die beiden Haarsträhnen mit Wasser gespült und anschließend mit 0,1 g eines Oxidationsmittels der folgenden Zusammensetzung nachbehandelt.

6,0 g Natriumbromat
1,0 g Dinatriumhydrogenphosphat
0,7 g Natriumdihydrogenphosphat
92,3 g Wasser
100,0 g (pH = 6,2)

Zu Vergleichszwecken wurden bei einer zweiten Testreihe die Haarsträhnen in der vorgenannten Weise verformt, wobei jedoch auf eine oxidative Nachbehandlung verzichtet wurde.

Anschließend wurden die Haarsträhnen mit Wasser gespült und getrocknet. Sodann wurde die Haltbarkeit der so erzeugten Locken ("Wellstabilität") durch Eintauchen der Haarsträhnen in Wasser geprüft.

Die Wellstabilität wurde mit Hilfe der folgenden Formel aus der gemessenen Länge der Haarsträhne nach der Verformungsbehandlung und der gemessenen Länge der Haarsträhne nach dem Aushängen in Wasser ermittelt.

$$\text{Wellstabilität } [\%] = \left( 1 - \frac{L2 - L1}{L_0 - L_1} \right) \times 100$$

$L_0$ = Länge der Haarsträhne vor der Verformung
$L_1$ = Länge der Haarsträhne nach der Verformung
$L_2$ = Länge der Haarsträhne nach dem Aushängen in Wasser

Je größer der Wert für die Wellstabilität ist, umso haltbarer ist die erzielte Dauerwelle. Das Ergebnis dieser Vergleichsversuche ist in der nachfolgenden Tabelle 1 zusammengefaßt.

Tabelle I

| verwendetes Verformungsmittel | oxidative Nachbehandlung | Wellstabilität % |
|---|---|---|
| (A) | ja | 19,2 |
| (A) | nein | 16,1 |
| (B) | ja | 25,4 |
| (B) | nein | 13,2 |

Die vorstehenden Vergleichsversuche zeigen, daß bei dem erfindungsgemäßen Verfahren durch die zusätzliche oxidative Nachbehandlung eine Dauerwellung erhalten wird, die gegenüber dem aus der EP-OS 0 346 151 bekannten Verfahren eine bis zu doppelt so gute Haltbarkeit aufweist.

**(b) Haltbarkeit der erzeugten Dauerwelle in Abhängigkeit vom pH-Wert des verwendeten Verformungsmittels**

Hierzu wurde die Haarsträhne mit einem Haarverformungsmittel der folgenden Zusammensetzung in der unter (a) beschriebenen Weise behandelt, wobei der pH-Wert des Haarverformungsmittels zwischen 7 und 10 variiert wurde.

**Haarverformungsmittel**

| | |
|---|---|
| Bunte-Salze der Formel (III), erhalten durch Umsetzung eines Polyamid-Epichlorhydrins ("Hercosett® 125" der Firma Hercules Inc., Wilmington/USA) mit Natriumthiosulfat | 10,0 g |
| Natriumsulfit x 7 $H_2O$ | 12,0 g |
| Natronlauge | ad pH 7 - 10 |
| Wasser | ad 100,0 g |

Anschließend wurde die Haarsträhne wie unter (a) beschrieben oxidativ nachbehandelt und getrocknet. Zu Vergleichszwecken wurde jeweils eine zweite Haarsträhne in gleicher Weise verformt, wobei jedoch auf eine oxidative Nachbehandlung verzichtet wurde.

Die Bestimmung der Wellstabilität erfolgte in der unter (a) beschriebenen Weise. Das Ergebnis dieser Untersuchung ist in Tabelle II zusammengefaßt.

Tabelle II

| pH-Wert des Haarverformungsmittels | Wellstabilität % | |
|---|---|---|
| | mit oxidativer Nachbehandlung | ohne oxidative Nachbehandlung |
| 7 | 40,4 | 33,8 |
| 8 | 40,8 | 35,8 |
| 9 | 37,0 | 35,8 |
| 10 | 37,7 | 34,6 |

Die vorstehenden Vergleichsversuche zeigen eindeutig, daß überraschenderweise bei dem erfindungsgemäßen Verfahren mit neutralen bis mildalkalischen Verformungsmitteln eine bessere und dauerhaftere Verformung bewirkt wird als bei Verwendung von stark alkalischen Verformungsmitteln.

## Patentansprüche

**1.** Verfahren zur dauerhaften Verformung von Haaren, bei dem man das Haar bevor und/oder nachdem man es in die gewünschte Form bringt mit einem Verformungsmittel behandelt, gegebenenfalls mit Wasser spült, sodann oxidativ nachbehandelt, mit Wasser spült, gegebenenfalls zur Wasserwelle legt, und sodann trocknet, dadurch gekennzeichnet, daß man als Verformungsmittel eine wäßrige Zubereitung, welche mindestens ein Sulfit oder Hydrogensulfit sowie mindestens ein Bunte-Salz enthält, verwendet.

**2.** Verfahren nach Anspruch 1, dadurch gekennzeichnet, daß das Haar vor der oxidativen Nachbehandlung getrocknet wird.

**3.** Verfahren nach Anspruch 1 oder 2, dadurch gekennzeichnet, daß das Verformungsmittel 0,1 bis 30 Gewichtsprozent mindestens eines Bunte-Salzes und 0,5 bis 30 Gewichtsprozent mindestens eines Sulfites oder Hydrogensulfites enthält.

**4.** Verfahren nach einem der Ansprüche 1 bis 3, dadurch gekennzeichnet, daß das Sulfit ausgewählt ist aus Alkalisulfiten oder -hydrogensulfiten, Ammoniumsulfit oder -hydrogensulfit und Alkanolaminsalzen der schwefeligen Säure.

**5.** Verfahren nach einem der Ansprüche 1 bis 4, dadurch gekennzeichnet, daß das Bunte-Salz ausgewählt ist aus

(i) 2-S-Thiosulfatocarbonsäuren der allgemeinen Formel (I)

$$M^{\oplus\ominus}OOC\text{-}(CH_2)_n\text{-}S_2O_3{}^{\ominus}M^{\oplus} \qquad \text{(I)},$$

mit n gleich einer ganzen Zahl von 1 bis 6 und $M^{\oplus}$ gleich einem Alkalimetall- oder Ammoniumion;

(ii) 2-S-Thiosulfatoethylamine der allgemeinen Formel (II)

$$H_2N\text{-}CH_2\text{-}CH_2\text{-}S_2O_3{}^{\ominus}M^{\oplus} \qquad \text{(II)},$$

mit $M^{\oplus}$ gleich einem Alkalimetall- oder Ammoniumion;

(iii) Polyamid-Epichlorhydrin-Bunte-Salze der allgemeinen Formel (III)

$$P\text{-}N\text{-}CH_2\text{-}CH(OH)\text{-}CH_2\text{-}S_2O_3{}^{\ominus}M^{\oplus} \qquad \text{(III)},$$

mit $M^{\oplus}$ gleich einem Alkalimetall- oder Ammoniumion und P = Polymerkette;

(iv) Polyoxyalkylen-Bunte-Salze der allgemeinen Formel (IV) oder (V)

$$\left[ R \left| \begin{array}{l} \left[(O\text{-}Alkylen)_m\ OH\right]_q \\ \\ \left[(O\text{-}Alkylen)_m\ O\ X\ S_2O_3Y\right]_p \end{array} \right. \right] \qquad \text{(IV)}$$

$$Y\ O_3S_2\ X\ \text{-}\ (O\text{-}Alkylen)_m\ \text{-}\ X\ S_2O_3Y \qquad \text{(V)}$$

wobei
p = 2, 3, 4, 5 oder 6;
q = 0, 1, 2, 3 oder 4;
(p + q) = 3, 4, 5 oder 6;
m gleich einer ganzen Zahl ≧ 1;
R einen Rest darstellt, der durch Abspalten der Hydroxylgruppen aus einem aliphatischen Alkohol mit mindestens 2 Kohlenstoffatomen erhalten wird;

X ein zweiwertiger substituierter oder unsubstituierter aliphatischer Rest mit 1 bis 10 Kohlenstoffatomen ist ; und

Y ein Wasserstoffatom, ein salzbildendes Ion oder eine salzbildende Gruppe darstellt;

(v) (3-S-Thiosulfato-2-hydroxy-propyl)-N,N-dimethylamin der allgemeinen Formel (VI)

$$(CH_3)_2N\text{-}CH_2\text{-}CH(OH)\text{-}CH_2\text{-}S_2O_3 \qquad \text{(VI)};$$

(vi) durch Umsetzung von Keratinhydrolysaten (beispielsweise Kerasol® der Firma Croda Ltd/Großbritannien) mit Natriumthiosulfat erhaltene Keratin-Bunte-Salze

(vii) Polysiloxan-Bunte-Salze der allgemeinen Formel (VII)

$$R^1_a\text{-}\underset{\displaystyle R^2_b}{\underset{|}{Si}}\text{-}O_{\frac{4-(a+b)}{2}} \qquad \text{(VII)}$$

wobei

$R^1$ = Methyl, jedoch bis zu 10 % der Gruppe Phenylreste sein können,

$R^2$ = $R^3$ und/oder $R^4$

mit

$R^3$ =

$$-(C_nH_{2n})-O-(C_mH_{2m}O)_p-R^5_q-CH_2\overset{\overset{\displaystyle R^7}{|}}{C}HCH_2R^6$$

$R^5$ =

$$\underset{\underset{\displaystyle CH_2Halogen}{|}}{-CH}-CH_2O- \quad \text{oder} \quad -CH_2-\underset{\underset{\displaystyle CH_2Halogen}{|}}{CH}-O-$$

$R^6$,$R^7$ = eine der beiden Gruppen -OH, die andere $-S_2O_3Me$
Me = Alkalimetall oder gegebenenfalls substituierter Ammoniumrest,
n = 3 bis 6,
m = 2 bis 3,
p = 0 bis 100,
q = 0 bis 5,
und
$R^4$ = $-(C_xH_{2x})-O-(C_yH_{2y}0)_zR^8$
$R^8$ = Wasserstoff, Alkylrest mit 1 bis 4 Kohlenstoffatomen oder Acetylrest,
x = 3 bis 6,
y = 2 bis 3,
z = 1 bis 100, ist,
mit der Maßgabe, daß im durchschnittlichen Molekül mindestens ein Rest $R^2$ die Bedeutung des Restes $R^3$ hat, und wenn im Rest $R^3$ p = 0 ist, mindestens 1 Rest $R^4$ vorhanden ist,
a = 1 bis 2,33
b = 0,02 bis 1 und
2 $\leqq (a + b) \leqq$ 3 ist.

**6.** Verfahren nach einem der Ansprüche 1 bis 5, dadurch gekennzeichnet, daß das Verformungsmittel einen pH-Wert von 6 bis 10 aufweist.

**7.** Verfahren nach einem der Ansprüche 1 bis 6, dadurch gekennzeichnet, daß man das Verformungsmittel 10 bis 60 Minuten lang einwirken läßt.

**8.** Verfahren nach einem der Ansprüche 1 bis 7, dadurch gekennzeichnet, daß das Verformungsmittel in einer Menge von 80 bis 120 Gramm angewendet wird.

**9.** Verfahren nach einem der Ansprüche 1 bis 8, dadurch gekennzeichnet, daß zur oxidativen Nachbehandlung eine 0,5 bis 10 Gewichtsprozent eines physiologisch verträglichen Oxidationsmittels enthaltende wäßrige Zubereitung verwendet wird.

**10.** Verfahren nach einem der Ansprüche 1 bis 9, dadurch gekennzeichnet, daß man das Oxidationsmittel 1 bis 20 Minuten einwirken läßt.

## Claims

**1.** Method of permanently shaping hair, in which, before and/or after the hair has been shaped in the desired style, it is treated with a shaping medium, optionally rinsed with water and then after-treated oxidatively, rinsed with water, if necessary set in a water wave and then dried, characterized in that an aqueous preparation containing at least one sulphite or hydrogen sulphite as well as at least one Bunte salt is used as the shaping medium.

**2.** Method according to Claim 1, characterized in that the hair is dried before the oxidative after-treatment.

**3.** Method according to Claim 1 or 2, characterized in that the shaping medium contains from 0.1 to 30 weight % of at least one Bunte salt and from 0.5 to 30 weight % of at least one sulphite or hydrogen sulphite.

**4.** Method according to any one of Claims 1 to 3, characterized in that the sulphite is selected from alkali sulphites or hydrogen sulphites, ammonium sulphite or hydrogen sulphite and alkanolamine salts of sulphuric acid.

**5.** Method according to any one of claims 1 to 4, characterized in that the Bunte salt is selected from:
(i) 2-S-thiosulphate carboxylic acids of the general formula (I)

$M^{\oplus\ominus}OOC\text{-}(CH_2)_n\text{-}S_2O_3{}^{\ominus}M^{\oplus}$ (I),

in which n is an integer from 1 to 6 and $M^{\oplus}$ is an alkali metal or ammonium ion;
(ii) 2-S-thiosulphate ethylamines of the general formula (II)

$H_2N\text{-}CH_2\text{-}CH_2\text{-}S_2O_3{}^{\ominus}\,M^{\oplus}$ (II),

in which $M^{\oplus}$ is an alkali metal or ammonium ion;
(iii) polyamide-epichlorohydrin Bunte salts of the general formula (III)

$P\text{-}N\text{-}CH_2\text{-}CH(OH)\text{-}CH_2\text{-}S_2O_3{}^{\ominus}\,M^{\oplus}$ (III),

in which $M^{\oplus}$ is an alkali metal or ammonium ion and P is a polymer chain;
(iv) polyoxyalkylene Bunte salts of the general formula (IV) or (V)

$$\left[R\right]\left[\begin{array}{l}\left[(O\text{-}Alkylen)_m\ OH\right]_q\\ \left[(O\text{-}Alkylen)_m\ O\ X\ S_2O_3Y\right]_p\end{array}\right.\qquad (IV)$$

in which
p = 2, 3, 4, 5 or 6;
q = 0, 1, 2, 3 or 4;
(p + q) = 3, 4, 5 or 6;
m is an integer ≧ 1;
R is a residue obtained by cleaving the hydroxyl groups from an aliphatic alcohol having at least two carbon atoms;
X is a bivalent substituted or non-substituted aliphatic radical with 1 to 10 carbon atoms; and
Y is a hydrogen atom, a salt-forming ion or a salt-forming group;
(v) (3-S-thiosulphato-2-hydroxy-propyl)-N,N-dimethylamine of the general formula (VI)

$(CH_3)_2N\text{-}CH_2\text{-}CH(OH)\text{-}CH_2\text{-}S_2O_3$ (VI);

(vi) by converting keratin hydrolysates (for example Kerasol® of Croda Ltd, Great Britain) with keratin Bunte salts obtained from sodium thiosulphate;

(vii) polysiloxane Bunte salts of the general formula (VII)

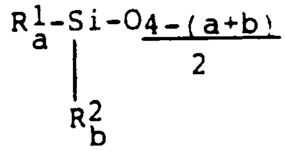 (VII)

in which
R[1] = methyl, but of which up to 10% may be the phenyl residue group
$R^2$ = $R^3$ and/or $R^4$
where
$R^3$ =

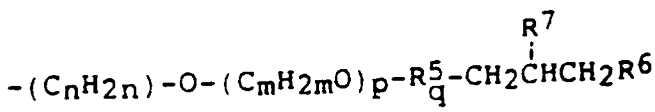

$R^5$ =

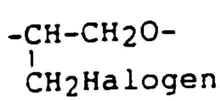 oder 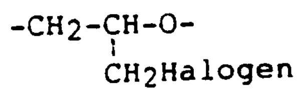

$R^6$, $R^7$ = one represents one of the two -OH groups and the other represents $-S_2O_3Me$
Me = alkali metal or optionally a substituted ammonium residue
n = 3 to 6
m = 2 to 3
p = 0 to 100
q = 0 to 5
and
$R^4$ = $-(C_xH_{2x})-0-(C_yH_{2y}0)_zR^8$
$R^8$ = hydrogen, alkyl residue with 1 to 4 carbon atoms or acetyl residue
x = 3 to 6
y = 2 to 3
z = 1 to 100,
provided that in the average molecule at least one $R^2$ residue has the significance of the $R^3$ residue and there is at least one $R^4$ residue in the residue if $R^3$ p = 0,
a = 1 to 2.33
b = 0.02 to 1 and
2 $\leqq (a + b) \leqq 3$.

**6.** Method according to any one of Claims 1 to 5, characterized in that the shaping medium has a pH of between 6 and 10.

**7.** Method according to any one of Claims 1 to 6, characterized in that the shaping medium is allowed to react for between 10 and 60 minutes.

**8.** Method according to any one of Claims 1 to 7, characterized in that the shaping medium is used in an amount of between 80 and 120 g.

9. Method according to any one of Claims 1 to 8, characterized in that an aqueous preparation containing from 0.5 to 10 weight % of a physiologically compatible oxidizing agent is used for the oxidative after-treatment.

10. Method according to any one of Claims 1 to 9, characterized in that the oxidizing agent is allowed to react for between 1 and 20 minutes.

**Revendications**

1. Procédé de mise en pli permanente des cheveux, selon lequel on traite les cheveux par un produit de mise en forme, avant et/ou après qu'ils aient été mis sous la forme souhaitée, on les rince le cas échéant avec de l'eau et on leur fait subir ensuite un traitement oxydant, puis un rinçage à l'eau, éventuellement en les mettant en pli, et ensuite un séchage, caractérisé en ce que comme agent de mise en pli, on utilise une préparation aqueuse qui contient au moins un sulfite ou un hydrosulfite ainsi qu'au moins un composé organique de thiosulfate..

2. Procédé selon la revendication 1, caractérisé en ce qu'on sèche les cheveux avant le post-traitement oxydant.

3. Procédé selon la revendication 1 ou 2, caractérisé en ce que l'agent de mise en pli contient de 0,1 à 30% en poids d'au moins un composé organique de thiosulfate et de 0,5 à 30% en poids d'au moins un sulfite ou un hydrosulfite.

4. Procédé selon l'une des revendications 1 à 3, caractérisé en ce que le sulfite est choisi parmi les sulfites ou les hydrosulfites alcalins, le sulfite ou l'hydrosulfite d'ammonium et les sels d'alcanolamine de l'acide sulfureux.

5. Procédé selon l'une des revendications 1 à 4, caractérisé en ce que le composé organique de thisulfate est choisi parmi:
(i) les acides 2-S-thiosulfato-carboxyliques de formule générale (I)

$M^{(+)(-)}OOC\text{-}(CH_2)_nS_2O_3{}^{(-)}M^{(+)}$ (I)

n étant un nombre entier allant de 1 à 6, et $M^{(+)}$ représentant un métal alcalin ou un ion ammonium;
(ii) des 2-S-thiosulfato-éthylamine de formule générale (II)

$H_2N\text{-}CH_2\text{-}CH_2\text{-}S_2O_3{}^{(-)}\ M^{(+)}$ (II)

avec $M^{(+)}$ représentant un métal alcalin ou un ion ammonium;
(iii) des composés organiques de thiosulfate polyamideépichlorhydrine de formule générale (III)

$P\text{-}N\text{-}CH_2\text{-}CH(OH)\text{-}CH_2\text{-}S_2O_3{}^{(+)}M^{(+)}$ (III)

dans laquelle $M^{(+)}$ représente un métal alcalin ou un ion ammonium et P = une chaîne de polymère;
(iv) des composés organiques de thiosulfate de polyoxyalkyle de formule générale iv ou v.

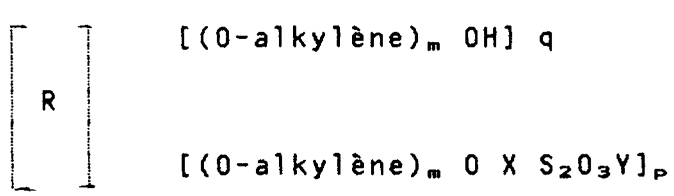

(IV)

$YO_3S_2X\text{-}(\ O\text{-}alkylène)_m\text{-}XS_2O_3Y$ (V)

dans lesquelles :

p = 2, 3, 4, 5 ou 6
q = 0, 1, 2, 3 ou 4
(p + q) = 3, 4, 5 ou 6
m est un nombre entier ≧ 1
R représente un reste qui est obtenu par dissociation des groupes hydroxyles d'un alcool aliphatique contenant au moins deux atomes de carbone, et
X est un reste aliphatique bivalent substitué ou non substitué contenant de 1 à 10 atomes de carbone;
Y représente un atome d'hydrogène, un ion formant un sel, ou des groupes formant des sels;
(v)(3-S-thiosulfato-2-hydroxy-propyl)-N-N-diméthylamine de formule générale (VI)

$(CH_3)N-CH_2-CH(OH)-CH_2-S_2O_3$ (VI)

(vi) des sels de keratine thiosulfato produit par réaction d'hydrolysats de kératine (par exemple kerasol(R) de la société Croda Ltd Grande Bretagne) avec du thiosulfate de sodium
(vii) des sels de thiosulfato-polysiloxane de formule générale (VII)

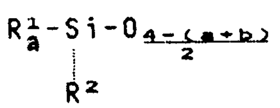 (VII)

dans laquelle
$R^1$ = méthyle, cependant jusqu'à 10% des groupes peuvent être des restes phényle
$R^2$ = $R^3$ et/ou $R^4$ avec
$R^3$ =

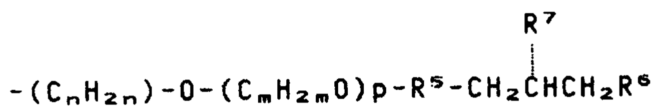

R5 =

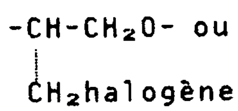 ou 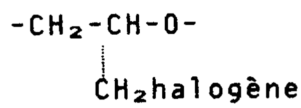

$R^6$, $R^7$ = un des deux représente -OH, l'autre $-S_2O_3Me$
Me = un métal alcalin ou un reste ammonium éventuellement substitué
n = 3 à 6
m = 2 à 3
p = 0 à 100
q = 0 à 5
et
R4 = $-(C_xH2_x)-O-(C_yH2_yO)_zR^8$
$R^8$ = hydrogène, un reste alkyle avec 1 à 4 atomes de carbone ou un reste acétyle
x = 3 à 6
y = 2 à 3
z = 1 à 100
avec la condition que dans la molécule moyenne au moins un reste $R^2$ ait la signification $R^3$ et quand dans le reste $R^3$p = 0, il y a au moins un reste $R^4$.
a = 1 à 2,33
b = 0,02 à 1 et
2 ≦ (a + b) ≦ 3

**6.** Procédé selon l'une des revendications 1 à 5, caractérisé en ce que l'agent de mise en pli présente une valeur de pH de 6 à 10.

**7.** Procédé selon l'une des revendications 1 à 6, caractérisé en ce qu'on laisse agir le produit pendant 10 à 60 minutes.

**8.** Procédé selon l'une des revendications 1 à 7, caractérisé en ce l'agent de mise en pli est utilisé à raison de 80 à 120 g.

**9.** Procédé selon l'une des revendications 1 à 8, caractérisé en ce que pour le post-traitement oxydant, on utilise une préparation aqueuse contenant des agents d'oxydation compatibles sur le plan physiologique, à raison de 0,05 à 10% en poids.

**10.** Procédé selon une des revendications 1 à 9, caractérisé en ce qu'on laisse agir l'agent d'oxydation pendant 1 à 20 minutes.